# EUROPEAN PATENT SPECIFICATION

(11) **EP 4 603 130 B1**
(45) Date of publication and mention of the grant of the patent: **02.12.2026**
(21) Application number: 25187035.8
(22) Date of filing: 21.07.2021
(51) Int. Cl.: A61M 5/162, A61M 5/24, A61M 39/10

(54) **DRUG DELIVERY DEVICE**
ARZNEIMITTELABGABEVORRICHTUNG
DISPOSITIF D'ADMINISTRATION DE MÉDICAMENT

(30) Priority: 22.07.2020 US 202063054850 P
(43) Date of publication of application: 20.08.2025
(60) Divisional application: 26213557.7
(62) Document number(s) of the earlier application(s) in accordance with Art. 76 EPC: 21755196.9 / 4 185 350
(73) Proprietor: Becton, Dickinson and Company, Franklin Lakes, NJ 07417 (US)
(72) Inventor: RABOLLI, Christina, Mahwah, NJ 07430 (US)
(74) Representative: Germain Maureau

(56) References cited:
- WO-A1-2020/243387
- CA-A1- 3 020 337
- US-A1- 2017 028 132

## Description

### CROSS-REFERENCE TO RELATED APPLICATIONS

The present application claims priority to United States Provisional Application Serial No. 63/054,850, entitled filed July 22, 2020.

### BACKGROUND OF THE INVENTION

### Field of the Invention

The disclosure relates generally to a drug delivery device and, in particular, to a valve assembly for a drug delivery device.

### Description of Related Art

Various types of automatic injection or drug delivery devices have been developed to allow drug solutions and other liquid therapeutic preparations to be administered by untrained personnel or to be self-injected. Generally, these devices include a reservoir that is pre-filled with the liquid therapeutic preparation, and some type of automatic needle-injection mechanism that can be triggered by the user. When the volume of fluid or drug to be administered is generally below a certain volume, such as 1 mL, an auto-injector is typically used, which typically has an injection time of about 10 to 15 seconds. When the volume of fluid or drug to be administered is above 1 mL, the injection time generally becomes longer resulting in difficulties for the patient to maintain contact between the device and the target area of the patient's skin. Further, as the volume of drug to be administered becomes larger, increasing the time period for injection becomes desirable. The traditional method for a drug to be injected slowly into a patient is to initiate an IV and inject the drug into the patient's body slowly. Such a procedure is typically performed in a hospital or outpatient setting.

Certain devices allow for self-injection in a home setting and are capable of gradually injecting a liquid therapeutic preparation into the skin of a patient. In some cases, these devices are small enough (both in height and in overall size) to allow them to be "worn" by a patient while the liquid therapeutic preparation is being infused into the patient. These devices typically include a pump or other type of discharge mechanism to force the liquid therapeutic preparation to flow out of a reservoir and into the injection needle. Such devices also typically include a valve or flow control mechanism to cause the liquid therapeutic preparation to begin to flow at the proper time and a triggering mechanism to initiate the injection.
A medicament device is disclosed in US 2017/028132.

### SUMMARY OF THE INVENTION

In one aspect or embodiment, it is provided a valve assembly for a drug delivery device according to claim 1.

The piercing member may be entirely spaced from the valve boot. The second end of the body of the piercing member is engaged with the valve sleeve. The second end of the body of the piercing member may received by a recessed area defined by the valve sleeve.

The valve sleeve may include a first cylindrical portion having a convex tip, a second portion extending from the first portion, and a third frustoconical portion extending from the second portion. The second end of the body of the piercing member may be received by a recessed area defined by the second portion of the valve sleeve. The first cylindrical portion of the valve sleeve may be positioned between the first end and the second end of the body of the piercing member. The body of the piercing member may be cylindrical.

The body of the piercing member may define a central passageway, with the central passageway receiving a portion of the valve sleeve. The valve sleeve may be formed from an elastomeric material. The second portion of the valve sleeve may include a frustoconical section and a cylindrical section. The third portion of the valve sleeve may include at least one recessed portion configured to facilitate a collapse and deformation of the valve sleeve. The valve boot may be formed from an elastomeric material. The piercing member may be formed from metal.

In further aspect or embodiment, a drug delivery device includes a housing, a cartridge received within the housing, with the cartridge configured to receive a medicament, a drive assembly received within the housing and configured to engage the cartridge and dispense medicament from the cartridge, a needle actuator assembly received within the housing, with the needle actuator assembly including a patient needle configured to pierce a patient's skin, and a valve assembly as described in any of the aspects or embodiments discussed above.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
FIG. 1 is a perspective view of a drug delivery system according to one aspect or embodiment of the present application.
FIG. 2 is a perspective, cross-sectional view of the drug delivery system of FIG. 1.
FIG. 3 is a front, cross-sectional view of the drug delivery system of FIG. 1.
FIG. 4 is a top view of the drug delivery system of FIG. 1, showing a top portion of the housing removed and the drug delivery system in a pre-use position.
FIG. 5 is a top, cross-sectional view of the drug delivery system of FIG. 1, showing the drug delivery system in a pre-use position.
FIG. 6 is a front, cross-sectional view of the drug delivery system of FIG. 1, showing the drug delivery system in a pre-use position.
FIG. 7 is a top view of the drug delivery system of FIG. 1, showing a top portion of the housing removed and the drug delivery system in an initial actuation position.
FIG. 8 is a top, cross-sectional view of the drug delivery system of FIG. 1, showing the drug delivery system in an initial actuation position.
FIG. 9 is a front, cross-sectional view of the drug delivery system of FIG. 1, showing the drug delivery system in an initial actuation position.
FIG. 10 is a top view of the drug delivery system of FIG. 1, showing a top portion of the housing removed and the drug delivery system in a use position.
FIG. 11 is a top, cross-sectional view of the drug delivery system of FIG. 1, showing the drug delivery system in a use position.
FIG. 12 is a front, cross-sectional view of the drug delivery system of FIG. 1, showing the drug delivery system in a use position.
FIG. 13 is a top view of the drug delivery system of FIG. 1, showing a top portion of the housing removed and the drug delivery system in a post-use position.
FIG. 14 is a top, cross-sectional view of the drug delivery system of FIG. 1, showing the drug delivery system in a post-use position.
FIG. 15A is a front, cross-sectional view of the drug delivery system of FIG. 1, showing the drug delivery system in a post-use position.
FIG. 15B is a front, cross-sectional view of the drug delivery system of FIG. 1, showing a pad with the drug delivery system in a pre-use position.
FIG. 15C is a perspective, cross-sectional view of the drug delivery system of FIG. 1, showing a pad with the drug delivery system in a pre-use position.
FIG. 15D is a perspective, cross-sectional view of the drug delivery system of FIG. 1, showing a pad with the drug delivery system in a pre-use position.
FIG. 16 is a partial cross-sectional view of the drug delivery system of FIG. 1, showing a valve assembly.
FIG. 17 is a cross-sectional view of a valve assembly of the drug delivery system of FIG. 1.
FIG. 18 is a perspective view of a valve sleeve of the valve assembly of the drug delivery system of FIG. 1.
FIG. 19 is a perspective view of a pierce member of the valve assembly of the drug delivery system of FIG. 1.

### DETAILED DESCRIPTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

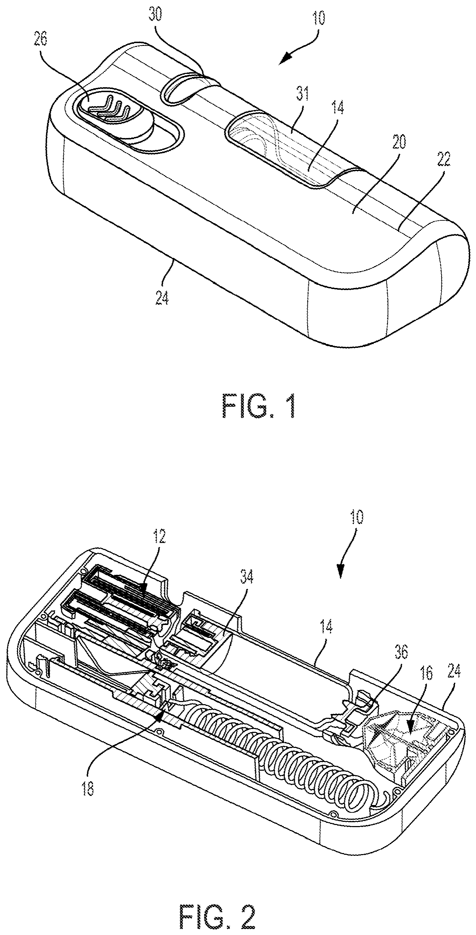

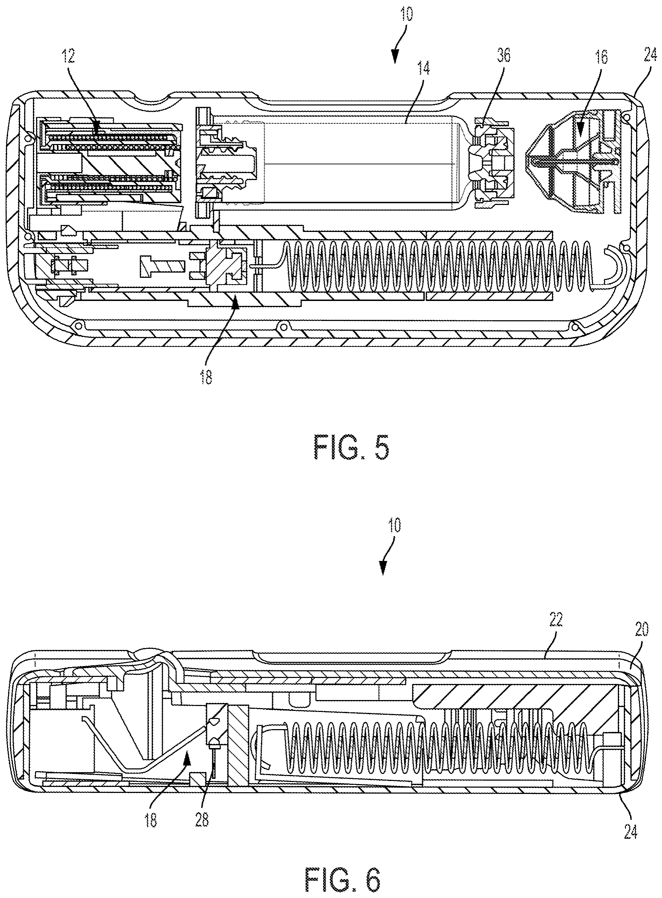

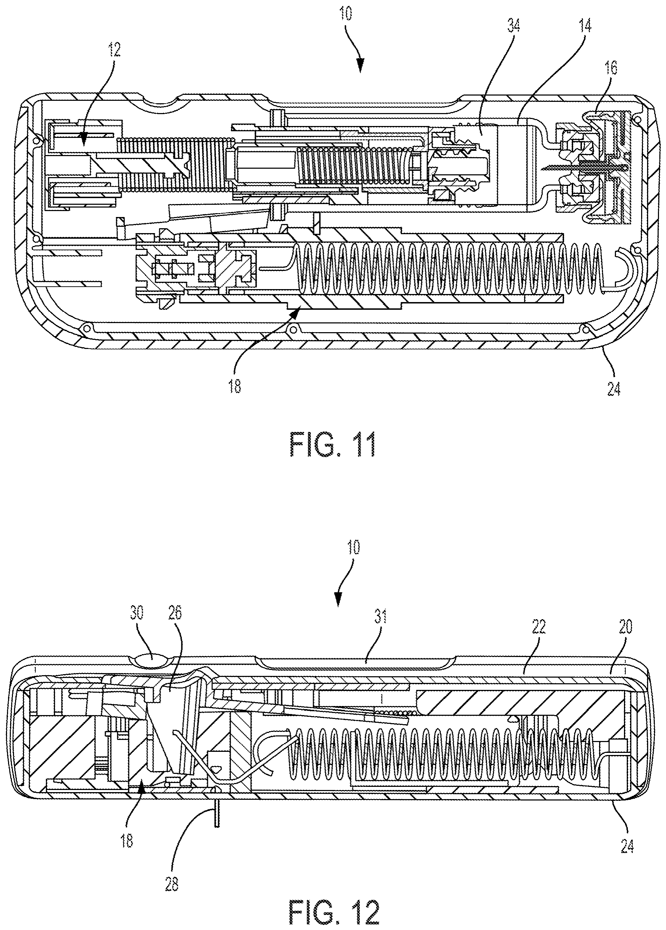

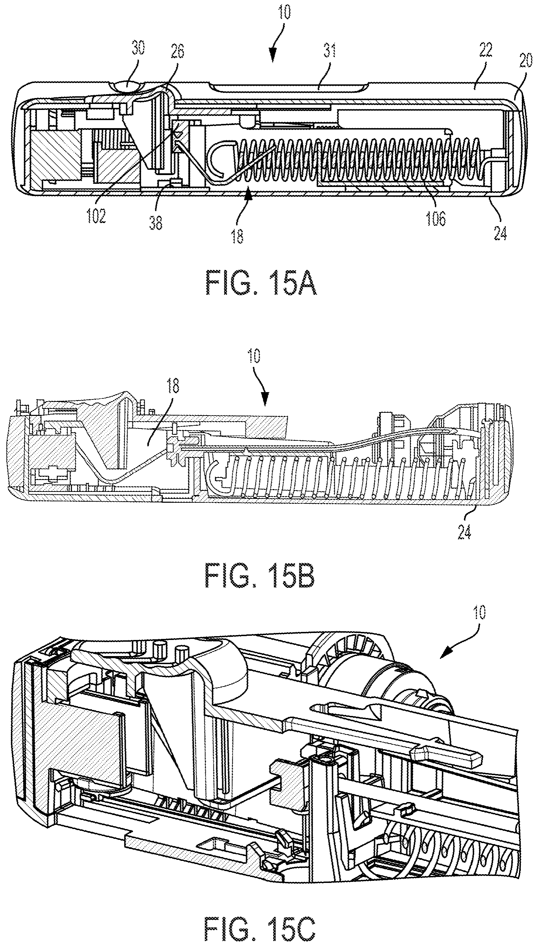

Referring to FIGS. 1-16, a drug delivery system 10 includes a drive assembly 12, a container 14, a valve assembly 16, and a needle actuator assembly 18. The drive assembly 12, the container 14, the valve assembly 16, and the needle actuator assembly 18 are at least partially positioned within a housing 20. The housing 20 includes a top portion 22 and a bottom portion 24, although other suitable arrangements for the housing 20 may be utilized. In one aspect, the drug delivery system 10 is an injector device configured to be worn or secured to a user and to deliver a predetermined dose of a medicament provided within the container 14 via injection into the user. The system 10 may be utilized to deliver a "bolus injection" where a medicament is delivered within a set time period. The medicament may be delivered over a time period of up to 45 minutes, although other suitable injection amounts and durations may be utilized. A bolus administration or delivery can be carried out with rate controlling or have no specific rate controlling. The system 10 may deliver the medicament at a fixed pressure to the user with the rate being variable. The general operation of the system 10 is described below in reference to FIGS. 1-16.

Referring again to FIGS. 1-16, the system 10 is configured to operate through the engagement of an actuation button 26 by a user, which results in a needle 28 of the needle assembly 18 piercing the skin of a user, the actuation of the drive assembly 12 to place the needle 28 in fluid communication with the container 14 and to expel fluid or medicament from the container 14, and the withdrawal of the needle 28 after injection of the medicament is complete. The general operation of a drug delivery system is shown and described in International Publication Nos. 2013/155153 and 2014/179774. The operation of the system 10 is also shown and described in U.S. Publication No. 2017/0354788. The housing 20 of the system 10 includes an indicator window 30 for viewing an indicator arrangement 32 configured to provide an indication to a user on the status of the system 10 and a container window 31 for viewing the container 14. The indicator window 30 may be a magnifying lens for providing a clear view of the indicator arrangement 32. The indicator arrangement 32 moves along with the needle actuator assembly 18 during use of the system 10 to indicate a pre-use status, use status, and post-use status of the system 10. The indicator arrangement 32 provides visual indicia regarding the status, although other suitable indicia, such an auditory or tactile, may be provided as an alternative or additional indicia.

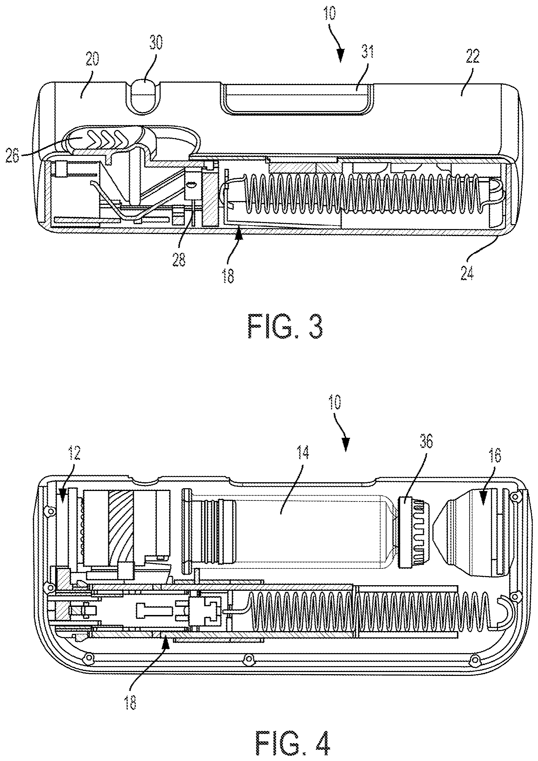

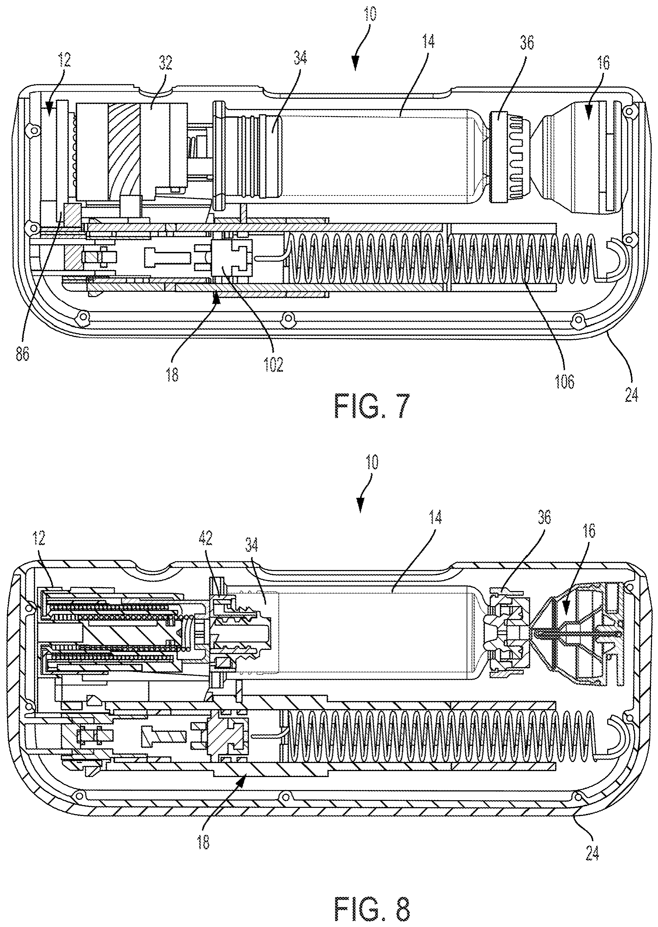

Referring to FIGS. 4-6, during a pre-use position of the system 10, the container 14 is spaced from the drive assembly 12, and the valve assembly 16 and the needle 28 is in a retracted position. During the initial actuation of the system 10, as shown in FIGS. 7-9, the drive assembly 12 engages the container 14 to move the container 14 toward the valve assembly 16, which is configured to pierce a closure 36 of the container 14 and place the medicament within the container 14 in fluid communication with the needle 28 via a tube (not shown) or other suitable arrangement. The drive assembly 12 is configured to engage a stopper 34 of the container 14, which will initially move the entire container 14 into engagement with the valve assembly 16 due to the incompressibility of the fluid or medicament within the container 14. The initial actuation of the system 10 is caused by engagement of the actuation button 26 by a user, which releases the needle actuator assembly 18 and the drive assembly 12 as discussed below in more detail. During the initial actuation, the needle 28 is still in the retracted position and about to move to the extended position to inject the user of the system 10.

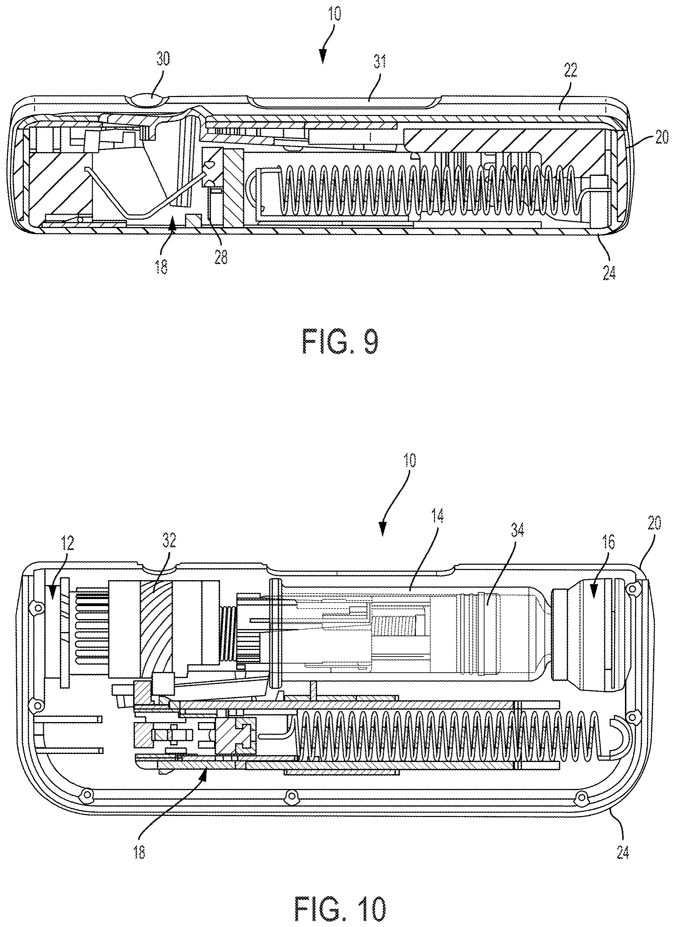

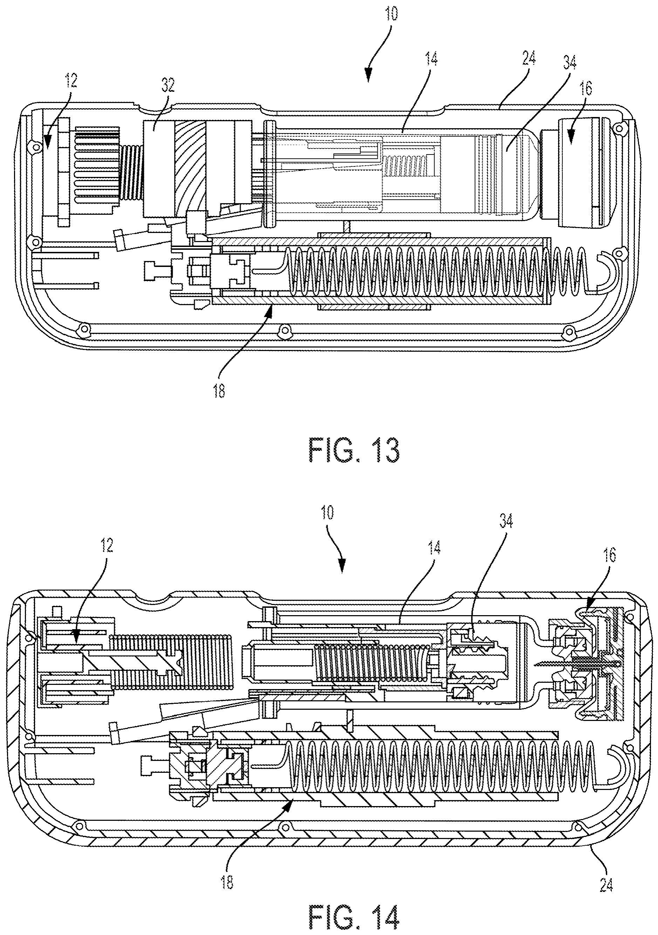

During the use position of the system 10, as shown in FIGS. 10-12, the needle 28 is in the extended position at least partially outside of the housing 20 with the drive assembly 12 moving the stopper 34 within the container 14 to deliver the medicament from the container 14, through the needle 28, and to the user. In the use position, the valve assembly 16 has already pierced a closure 36 of the container 14 to place the container 14 in fluid communication with the needle 28, which also allows the drive assembly 12 to move the stopper 34 relative to the container 14 since fluid is able to be dispensed from the container 14. At the post-use position of the system 10, shown in FIGS. 13-15, the needle 28 is in the retracted position and engaged with a pad 38 to seal the needle 28 and prevent any residual flow of fluid or medicament from the container 14.

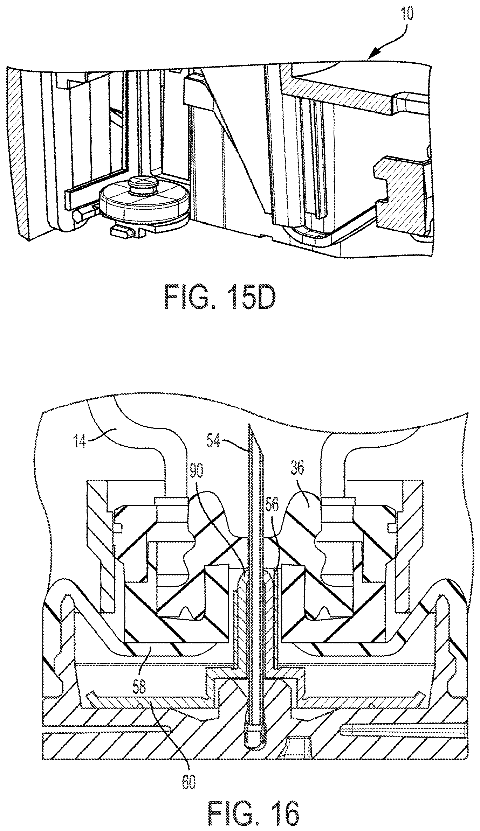

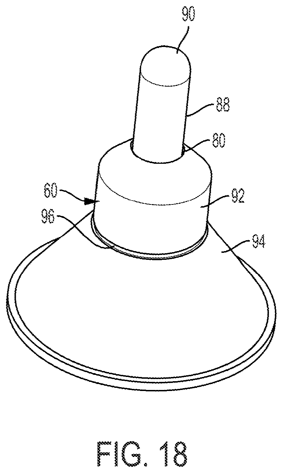

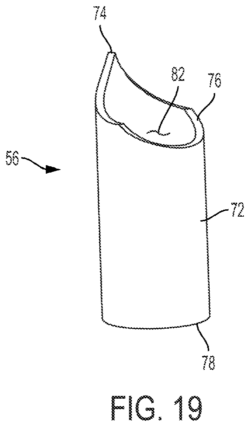

Referring to FIGS. 16-19, as discussed above, the valve assembly 16 operates to facilitate fluid communication between the container 14 and the needle actuator assembly 18. The valve assembly 16 includes a valve housing 52, a cannula 54, a piercing member 56, and a valve boot 58. The valve assembly 16 also includes a valve sleeve 60. The valve housing 52 has a first side 62 and a second side 64 positioned opposite from the first side 62. The valve housing 52 may be formed integrally with the housing 20 of the system 10 or may be formed as a separate component. The cannula 54 has a first end 66 and a second end 68 positioned opposite from the first end 66. The cannula 54 defines a central passageway 70. The first end 66 of the cannula 54 is sharp and configured to pierce a septum of the container 14. The second end 68 of the cannula 54 is received by and secured to the valve housing 52. The valve housing 52 is in fluid communication with the needle actuator assembly 18 via tubing (not shown) to form a fluid flow path from the cannula 54 to the needle actuator assembly 18.

Referring again to FIGS. 16-19, the piercing member 56 includes a body 72 with a piercing tip 74 extending from the body 72. The body 72 of the piercing member 56 has a first end 76 and a second end 78 positioned opposite the first end 76. The piercing tip 74 extends from the first end 76 of the body 72. The piercing member 56 is engaged with the valve sleeve 60. The piercing member 56 is entirely spaced from the valve boot 58. In one aspect or embodiment, the piercing member 56 is not directly or indirectly in contact with the valve boot 58. The second end 78 of the body 72 of the piercing member 56 is engaged with the valve sleeve 60. In one aspect or embodiment, the second end 78 of the body 72 is received by a recessed area 80 defined by the valve sleeve 60. The body 72 of the piercing member 56 is cylindrical, although other suitable shapes and configurations may be utilized. The body 72 of the piercing member 56 defines a central passageway 82 that receives a portion of the valve sleeve 60. In one aspect or embodiment, the piercing member 56 is snugly positioned over the valve sleeve 60 with the piercing member 56 in contact with the valve sleeve 60 from the first end 76 of the body 72 to the second end 78 of the body 72. The piercing member 56 is formed from metal, although other suitable materials may be utilized. The piercing member 56 may be manufactured using a stamping, rolling, and/or forming process.

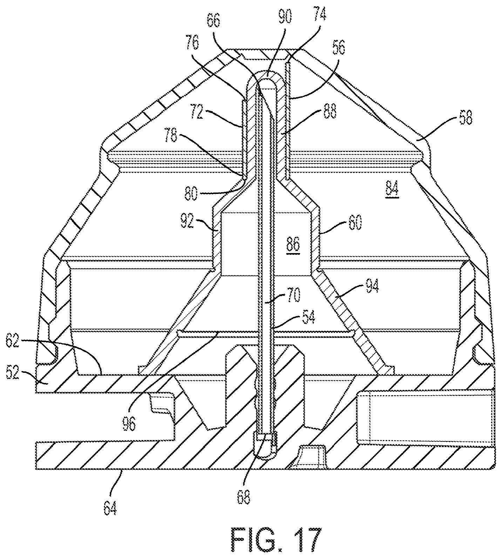

The valve boot 58 is connected to the valve housing 52 and defines an interior space 84. The valve boot 58 may be formed from an elastomeric material, although other suitable materials or combination of materials may be utilized. The valve boot 58 is configured to move from a pre-use position where the first end 66 of the cannula 54 and the piercing tip 74 of the piercing member 56 are received within the interior space 84 to a use position where the piercing tip 74 of the piercing member 56 and the first end 66 of the cannula 54 extend outside of the valve boot 58 and the interior space 84. The pre-use position of the valve boot 58 and the valve assembly 16 is shown in FIG. 17. The use position of the valve boot 58 and the valve assembly 16 is shown in FIG. 16. In the use position, the piercing tip 74 pierces the valve boot 58 and a foil seal (not shown) on the container 14 and the cannula 54 pierces a septum of the container 14 to provide fluid communication with the interior of the container 14 and the piercing member 56 moving toward the valve housing 52.

Referring to FIGS. 16-18, the valve sleeve 60 is positioned within the interior space 84 defined by the valve boot 58. The valve sleeve 60 defines a cannula space 86 and is configured to move from a pre-use position where the first end 66 of the cannula 54 is received within the cannula space 86 to a use position where the first end 66 of the cannula 54 extends outside of the valve sleeve 60 and the cannula space 86. The valve sleeve 60 may be formed from an elastomeric material, such as a rubber material, although other suitable materials or combination of materials may also be utilized. The valve sleeve 60 includes a first cylindrical portion 88 having a convex tip 90, a second portion 92 extending from the first portion 88, and a third frustoconical portion 94 extending from the second portion 92. The second portion 92 is frustoconical and cylindrical. The third portion 94 of the valve sleeve 60 may include one or more recessed portions 96 to facilitate the collapse and deformation of the valve sleeve 60. Upon engagement of the valve assembly 16 with the container 14, the valve sleeve 60 will retract with the first end 66 of the cannula 54 extending through the valve sleeve 60.

The valve sleeve 60 is configured to ensure the cannula 54 and flow path of the system 10 remain free from contamination during operation of the system 10. During operation of the system 10, as discussed above, the container 14 is moved into engagement with the valve boot 58 to collapse the valve boot 58 with the piercing tip 74 of the piercing member 56 puncturing the foil seal of the container 14 and the valve boot 58 to form respective chads or flaps (not shown). The valve sleeve 60 prevents any contamination present on the container 14 and valve boot 58 from being discharged or flung from the container 14 or valve boot 58 onto or into the cannula 54. The valve sleeve 60 also shields the cannula 54 from contact with the chads or flaps formed by the piercing member 76 and minimizes the surface area of the closure 36 of the container 14 through which the cannula 54 must pass through. The valve sleeve 60 is also configured to prevent contamination caused by the depressurization of the valve boot 58 when the valve boot 58 is compressed and subsequently pierced by the piercing member 76 by preventing the settling of entrained contamination onto the external and internal surfaces of the cannula 54.

In one aspect or embodiment, the second end 78 of the body 72 of the piercing member 56 may be flared to allow the piercing member 56 to more easily slide over the first cylindrical portion 88 of the valve sleeve 60.

In one aspect or embodiment, a thickness of the valve sleeve 60 as well as angles of the first cylindrical portion 88, second portion 92, and third portion 94 may be varied to optimize a force required to move the valve sleeve from the pre-use position to the use position. The valve sleeve 60 may be configured to fully pierce the valve boot 58, as discussed above, before the valve sleeve 60 collapses or moves from the pre-use position to the use position.

Referring to FIG. 17, the second end 78 of the body 72 of the piercing member 56 is received in the recessed area 80 defined by the second portion 92 of the valve sleeve 60. The first cylindrical portion 88 of the valve sleeve 60 is positioned between the first end 76 and the second end 78 of the body 72 of the piercing member 56.

Elements of one disclosed aspect can be combined with elements of one or more other disclosed aspects to form different combinations, all of which are considered to be within the scope of the present invention.

While this disclosure has been described as having exemplary designs, the present disclosure can be further modified within the scope of this disclosure. This application is therefore intended to cover any variations, uses, or adaptations of the disclosure using its general principles. Further, this application is intended to cover such departures from the present disclosure as come within known or customary practice in the art to which this disclosure pertains and which fall within the limits of the appended claims.

## Claims

1. A valve assembly (16) for a drug delivery device, the valve assembly comprising:
a valve housing (52) having a first side (62) and a second side (64) positioned opposite from the first side (62),
a cannula (54) having a first end (66) and a second end (68) positioned opposite the first end (66), the cannula (54) defining a central passageway (70),
a valve boot (58) connected to the valve housing (52) and defining an interior space (84), the valve boot (58) is configured to move from a pre-use position where the first end (66) of the cannula (54) is received within the interior space (84) to a use position where the first end (66) of the cannula (54) extends outside of the valve boot (58) and the interior space (84),
a valve sleeve (60) defining a cannula space (86), the valve sleeve (60) being positioned within the interior space (84) defined by the valve boot (58),
a piercing member (56) having a body (72) with a piercing tip (74), the body (72) of the piercing member (56) having a first end (76) and a second end (78) positioned opposite the first end (76),
**characterized in that** the piercing member (56) is engaged with the valve sleeve (60), the body (72) of the piercing member (56) defining a central passageway (82) that receives a portion of the valve sleeve (60), and **in that** the valve sleeve (60) is configured to move from a pre-use position where the first end (66) of the cannula (54) is received within the cannula space (86) and the interior space (84) to a use position where the first end (66) of the cannula (54) extends outside of the valve sleeve (60) and the cannula space (86).

2. The valve assembly of claim 1, wherein the piercing member (56) is entirely spaced from the valve boot (58).

3. The valve assembly of claim 1, wherein the second end (78) of the body (72) of the piercing member (56) is engaged with the valve sleeve (60).

4. The valve assembly of claim 3, wherein the second end (78) of the body (72) of the piercing member (56) is received by a recessed area (80) defined by the valve sleeve (60).

5. The valve assembly of claim 1, wherein the valve sleeve (60) comprises a first cylindrical portion (88) having a convex tip (90), a second portion (92) extending from the first portion (88), and a third frustoconical portion (94) extending from the second portion (92).

6. The valve assembly of claim 5, wherein the second end (78) of the body (72) of the piercing member (56) is received by a recessed area (80) defined by the second portion (92) of the valve sleeve (60).

7. The valve assembly of claim 5, wherein the first cylindrical portion (88) of the valve sleeve (60) is positioned between the first end (76) and the second end (78) of the body (72) of the piercing member (56).

8. The valve assembly of claim 5, wherein the second portion (92) of the valve sleeve (60) comprises a frustoconical section and a cylindrical section.

9. The valve assembly of claim 5, wherein the third portion (94) of the valve sleeve (60) comprises at least one recessed portion (96) configured to facilitate a collapse and deformation of the valve sleeve (60).

10. The valve assembly of claim 1, wherein the body (72) of the piercing member (56) is cylindrical.

11. The valve assembly of claim 1, wherein the valve sleeve (60) comprises an elastomeric material.

12. The valve assembly of claim 1, wherein the valve boot (58) comprises an elastomeric material.

13. The valve assembly of claim 1, wherein the piercing member (56) comprises metal.

14. The valve assembly of claim 1, wherein the valve sleeve (60) is configured to fully pierce the valve boot (58) before the valve sleeve (60) moves from the pre-use position to the use position.

15. A drug delivery device (10) comprising:
a housing (20);
a cartridge (14) received within the housing (20), the cartridge (14) configured to receive a medicament;
a drive assembly (12) received within the housing (20) and configured to engage the cartridge (14) and dispense medicament from the cartridge (14);
a needle actuator assembly (18) received within the housing (20), the needle actuator assembly (18) comprising a patient needle (28) configured to pierce a patient's skin; and
a valve assembly (16) according to any of claims 1 to 14.

## Patentansprüche

1. Ventilbaugruppe (16) für eine Arzneimittelabgabevorrichtung, wobei die Ventilbaugruppe Folgendes umfasst:
ein Ventilgehäuse (52), das eine erste Seite (62) und eine zweite Seite (64), die gegenüber der ersten Seite (62) positioniert sind, aufweist,
eine Kanüle (54), die ein erstes Ende (66) und ein zweites Ende (68), das gegenüber dem ersten Ende (66) positioniert ist, aufweist, wobei die Kanüle (54) einen zentralen Durchgang (70) definiert,
eine Ventilmanschette (58), die mit dem Ventilgehäuse (52) verbunden ist und einen Innenraum (84) definiert, wobei die Ventilmanschette (58) so eingerichtet ist, dass sie sich von einer Vorgebrauchsposition, in der das erste Ende (66) der Kanüle (54) innerhalb des Innenraums (84) aufgenommen ist, in eine Gebrauchsposition bewegt, in der sich das erste Ende (66) der Kanüle (54) außerhalb der Ventilmanschette (58) und des Innenraums (84) erstreckt,
eine Ventilhülse (60), die einen Kanülenraum (86) definiert, wobei die Ventilhülse (60) innerhalb des von der Ventilmanschette (58) definierten Innenraums (84) positioniert ist,
ein Durchstichelement (56), das einen Körper (72) mit einer Durchstichspitze (74) aufweist, wobei der Körper (72) des Durchstichelements (56) ein erstes Ende (76) und ein zweites Ende (78), das gegenüber dem ersten Ende (76) positioniert ist, aufweist,
**dadurch gekennzeichnet, dass** das Durchstichelement (56) mit der Ventilhülse (60) in Eingriff steht, wobei der Körper (72) des Durchstichelements (56) einen zentralen Durchgang (82) definiert, der einen Teil der Ventilhülse (60) aufnimmt, und dass die Ventilhülse (60) so eingerichtet ist, dass sie sich von einer Vorgebrauchsposition, in der das erste Ende (66) der Kanüle (54) innerhalb des Kanülenraums (86) und des Innenraums (84) aufgenommen wird, zu einer Gebrauchsposition bewegt, in der sich das erste Ende (66) der Kanüle (54) außerhalb der Ventilhülse (60) und des Kanülenraums (86) erstreckt.

2. Ventilbaugruppe nach Anspruch 1, wobei das Durchstichelement (56) vollständig von der Ventilmanschette (58) beabstandet ist.

3. Ventilbaugruppe nach Anspruch 1, wobei das zweite Ende (78) des Körpers (72) des Durchstichelements (56) mit der Ventilhülse (60) in Eingriff steht.

4. Ventilbaugruppe nach Anspruch 3, wobei das zweite Ende (78) des Körpers (72) des Durchstichelements (56) von einem vertieften Bereich (80) aufgenommen wird, der durch die Ventilhülse (60) definiert ist.

5. Ventilbaugruppe nach Anspruch 1, wobei die Ventilhülse (60) einen ersten zylindrischen Abschnitt (88) umfasst, der eine konvexe Spitze (90), einen zweiten Abschnitt (92), der sich aus dem ersten Abschnitt (88) erstreckt, und einen dritten kegelstumpfförmigen Abschnitt (94), der sich aus dem zweiten Abschnitt (92) erstreckt, umfasst.

6. Ventilbaugruppe nach Anspruch 5, wobei das zweite Ende (78) des Körpers (72) des Durchstichelements (56) von einem vertieften Bereich (80) aufgenommen wird, der durch den zweiten Abschnitt (92) der Ventilhülse (60) definiert ist.

7. Ventilbaugruppe nach Anspruch 5, wobei der erste zylindrische Abschnitt (88) der Ventilhülse (60) zwischen dem ersten Ende (76) und dem zweiten Ende (78) des Körpers (72) des Durchstichelements (56) positioniert ist.

8. Ventilbaugruppe nach Anspruch 5, wobei der zweite Abschnitt (92) der Ventilhülse (60) einen kegelstumpfförmigen Abschnitt und einen zylindrischen Abschnitt umfasst.

9. Ventilbaugruppe nach Anspruch 5, wobei der dritte Abschnitt (94) der Ventilhülse (60) mindestens einen vertieften Abschnitt (96) umfasst, der so eingerichtet ist, dass er ein Zusammenfallen und eine Verformung der Ventilhülse (60) erleichtert.

10. Ventilbaugruppe nach Anspruch 1, wobei der Körper (72) des Durchstichelements (56) zylindrisch ist.

11. Ventilbaugruppe nach Anspruch 1, wobei die Ventilhülse (60) ein Elastomermaterial umfasst.

12. Ventilbaugruppe nach Anspruch 1, wobei die Ventilmanschette (58) ein Elastomermaterial umfasst.

13. Ventilbaugruppe nach Anspruch 1, wobei das Durchstichelement (56) Metall umfasst.

14. Ventilbaugruppe nach Anspruch 1, wobei die Ventilhülse (60) so eingerichtet ist, dass sie die Ventilmanschette (58) vollständig durchsticht, bevor sich die Ventilhülse (60) von der Vorgebrauchsposition in die Gebrauchsposition bewegt.

15. Arzneimittelabgabevorrichtung (10), umfassend:
ein Gehäuse (20);
eine Kartusche (14), die innerhalb des Gehäuses (20) aufgenommen wird, wobei die Kartusche (14) so eingerichtet ist, dass sie ein Medikament aufnimmt;
eine Antriebsbaugruppe (12), die innerhalb des Gehäuses (20) aufgenommen wird und so eingerichtet ist, dass sie die Kartusche (14) einrastet und das Medikament aus der Kartusche (14) abgibt;
eine Nadelbetätigungsbaugruppe (18), die innerhalb des Gehäuses (20) aufgenommen wird, wobei die Nadelbetätigungsbaugruppe (18) eine Patientennadel (28) umfasst, die so eingerichtet ist, dass sie die Haut eines Patienten durchsticht; und
eine Ventilbaugruppe (16) nach einem der Ansprüche 1 bis 14.

## Revendications

1. Ensemble soupape (16) pour un dispositif d'administration de médicament, l'ensemble soupape comprenant :
un boîtier de soupape (52) ayant un premier côté (62) et un second côté (64) positionné à l'opposé du premier côté (62),
une canule (54) ayant une première extrémité (66) et une seconde extrémité (68) positionnée à l'opposé de la première extrémité (66), la canule (54) définissant un passage central (70),
un soufflet de soupape (58) relié au boîtier de soupape (52) et définissant un espace intérieur (84), le soufflet de soupape (58) est configuré pour se déplacer d'une position de pré-utilisation, dans laquelle la première extrémité (66) de la canule (54) est reçue dans l'espace intérieur (84) à une position d'utilisation, dans laquelle la première extrémité (66) de la canule (54) s'étend à l'extérieur du soufflet de soupape (58) et de l'espace intérieur (84),
un manchon de soupape (60) définissant un espace de canule (86), le manchon de soupape (60) étant positionné dans l'espace intérieur (84) défini par le soufflet de soupape (58),
un élément de perçage (56) ayant un corps (72) avec une pointe de perçage (74), le corps (72) de l'élément de perçage (56) ayant une première extrémité (76) et une seconde extrémité (78) positionnée à l'opposé de la première extrémité (76),
**caractérisé en ce que** l'élément de perçage (56) est engagé avec le manchon de soupape (60), le corps (72) de l'élément de perçage (56) définissant un passage central (82) qui reçoit une partie du manchon de soupape (60), et **en ce que** le manchon de soupape (60) est configuré pour se déplacer d'une position de pré-utilisation, dans laquelle la première extrémité (66) de la canule (54) est reçue dans l'espace de canule (86) et l'espace intérieur (84), à une position d'utilisation, dans laquelle la première extrémité (66) de la canule (54) s'étend à l'extérieur du manchon de soupape (60) et de l'espace de canule (86).

2. Ensemble soupape selon la revendication 1, dans lequel l'élément de perçage (56) est entièrement espacé du soufflet de soupape (58).

3. Ensemble soupape selon la revendication 1, dans lequel la seconde extrémité (78) du corps (72) de l'élément de perçage (56) est engagée avec le manchon de soupape (60).

4. Ensemble soupape selon la revendication 3, dans lequel la seconde extrémité (78) du corps (72) de l'élément de perçage (56) est reçue par une zone évidée (80) définie par le manchon de soupape (60).

5. Ensemble soupape selon la revendication 1, dans lequel le manchon de soupape (60) comprend une première partie cylindrique (88) ayant une pointe convexe (90), une deuxième partie (92) s'étendant depuis la première partie (88), et une troisième partie tronconique (94) s'étendant depuis la deuxième partie (92).

6. Ensemble soupape selon la revendication 5, dans lequel la seconde extrémité (78) du corps (72) de l'élément de perçage (56) est reçue par une zone évidée (80) définie par la deuxième partie (92) du manchon de soupape (60).

7. Ensemble soupape selon la revendication 5, dans lequel la première partie cylindrique (88) du manchon de soupape (60) est positionnée entre la première extrémité (76) et la seconde extrémité (78) du corps (72) de l'élément de perçage (56).

8. Ensemble soupape selon la revendication 5, dans lequel la deuxième partie (92) du manchon de soupape (60) comprend une section tronconique et une section cylindrique.

9. Ensemble soupape selon la revendication 5, dans lequel la troisième partie (94) du manchon de soupape (60) comprend au moins une partie évidée (96) configurée pour faciliter l'affaissement et la déformation du manchon de soupape (60).

10. Ensemble soupape selon la revendication 1, dans lequel le corps (72) de l'élément de perçage (56) est cylindrique.

11. Ensemble soupape selon la revendication 1, dans lequel le manchon de soupape (60) comprend un matériau élastomère.

12. Ensemble soupape selon la revendication 1, dans lequel le soufflet de soupape (58) comprend un matériau élastomère.

13. Ensemble soupape selon la revendication 1, dans lequel l'élément de perçage (56) comprend du métal.

14. Ensemble soupape selon la revendication 1, dans lequel le manchon de soupape (60) est configuré pour percer entièrement le soufflet de soupape (58) avant que le manchon de soupape (60) ne se déplace de la position de pré-utilisation à la position d'utilisation.

15. Dispositif d'administration de médicament (10) comprenant :
un boîtier (20) ;
une cartouche (14) reçue à l'intérieur du boîtier (20), la cartouche (14) étant configurée pour recevoir un médicament ;
un ensemble d'entraînement (12) reçu à l'intérieur du boîtier (20) et configuré pour s'engager avec la cartouche (14) et distribuer le médicament à partir de la cartouche (14) ;
un ensemble d'actionnement d'aiguille (18) reçu à l'intérieur du boîtier (20), l'ensemble d'actionnement d'aiguille (18) comprenant une aiguille côté patient (28) configurée pour percer la peau d'un patient ; et
un ensemble soupape (16) selon l'une des revendications 1 à 14.
